# FASCICULE DE BREVET EUROPEEN

(11) **EP 2 335 699 B1**
(45) Date de publication et mention de la délivrance du brevet: **30.03.2016**
(21) Numéro de dépôt: 10193486.7
(22) Date de dépôt: 02.12.2010
(51) Int. Cl.: A61K 31/395, A61K 31/415, A61K 31/42, A61K 45/06, A61P 25/04

(54) **COMBINAISON SYNERGIQUE DE COMPOSÉS ANALGÉSIQUES**
SYNERGISTISCHE KOMBINATION VON SCHMERZMITTELN
SYNERGISTIC COMBINATION OF ANALGESIC COMPOUNDS

(30) Priorité: 03.12.2009 FR 0958610
(43) Date de publication de la demande: 22.06.2011
(73) Titulaire: Biocodex, 94250 Gentilly (FR)
(72) Inventeur: Girard, Philippe, 60280, MARGNY-LES-COMPIEGNE (FR); Le Guern, Marie-Emmanuelle, 60200, COMPIEGNE (FR); Gillardin, Jean-Marie, 60680, JONQUIERES (FR); Hublot, Bernard, 60200, COMPIEGNE (FR)
(74) Mandataire: Vial, Lionel

(56) Documents cités:
- MOFFAT A C ET AL: "POSTOPERATIVE NEFOPAM AND DICLOFENAC. EVALUATION OF THEIR MORPHINE-SPARING EFFECT AFTER UPPER ABDOMINAL SURGERY", ANAESTHESIA, ACADEMIC PRESS, LONDON, GB, vol. 45, no. 4, 1 janvier 1990 (1990-01-01), pages 302-305, XP009017736, ISSN: 0003-2409
- LASSETER K C ET AL: "NEFOPAN HCl INTERACTION STUDY WITH EIGHT OTHER DRUGS", JOURNAL OF INTERNATIONAL MEDICAL RESEARCH, CAMBRIDGE MEDICAL PUBLICATIONS LTD, GB, vol. 4, no. 3, 1 janvier 1976 (1976-01-01) , pages 195-201, XP009017739, ISSN: 0300-0605
- BENHAMOU D (REPRINT): "NEFOPAM AND COMBINED ANALGESICS// NEFOPAM ET ASSOCIATION D'ANALGESIQUES", ANNALES FRANCAISES D'ANESTHESIE ET DE REANIMATION, MASSON, PARIS, FR, 1 décembre 2002 (2002-12-01), pages 9-14, XP009017788, ISSN: 0750-7658
- DATABASE WPI Week 200950 Thomson Scientific, London, GB; AN 2009-H90449 XP002578588, & CN 101 411 702 A (UNIV HENAN) 22 avril 2009 (2009-04-22)

## Description

### Domaine de l'invention

La présente invention concerne une composition pharmaceutique comprenant des composés ayant une action synergique dans la prévention ou le traitement de la douleur.

### Arrière-plan technique

Au plan pharmacologique, les anti-inflammatoires non-stéroïdiens (AINS) agissent en inhibant une enzyme, la cyclooxygénase (COX), et donc la production de prostaglandines qui découle de son activité.

La COX existe chez l'homme sous 2 isoformes majeures qui sont impliquées dans des processus différents. La cyclooxygénase de type 1 (COX-1) intervient notamment dans le processus physiologique de protection gastrique tandis que la cyclooxygénase de type 2 (COX-2) est principalement impliquée dans le processus inflammatoire.

D'une manière générale, les AINS conventionnels, non-sélectifs, inhibent à la fois la COX-1 et la COX-2. En conséquence, leur utilisation est associée à des effets secondaires indésirables, à savoir, notamment, des lésions gastro-intestinales, qui découlent directement de l'inhibition de la COX-1.

Ceci a conduit au développement d'inhibiteurs sélectifs de la cyclooxygénase de type 2 (COX-2), comprenant la classe médicamenteuse (ou thérapeutique) des COXIBs, pour lesquels les effets gastro-intestinaux sont significativement diminués par rapport aux AINS non-sélectifs (Moore *et al.* (2006) *BMC Musculoskeletal Disorders* **7**:79-91). Les principales indications des COXIBs concernent les douleurs articulaires. On peut ainsi citer, à titre d'exemple, le célécoxib (Celebrex®), le parécoxib (Dynastat®), le valdécoxib (Bextra®), le rofécoxib (Vioxx®), l'étoricoxib (Arcoxia®), ou encore le lumiracoxib (Prexige®).

Toutefois, un des effets secondaires majeurs des COXIBs est l'apparition de troubles cardiovasculaires (voir par exemple Caldwell et al. (2006) J. R. Soc. Med. 99:132-140). De ce fait, à l'heure actuelle, le valdécoxib et le rofécoxib ont été retirés du marché et certains pays ont refusé d'autoriser la mise sur le marché du parécoxib. Par ailleurs, le lumiracoxib a également essuyé des refus de mise sur le marché du fait d'atteintes hépatiques qu'il provoquerait.

Il serait donc important de pouvoir bénéficier des avantages thérapeutiques offerts par les COXIBs tout en limitant leurs effets secondaires.

Le néfopam est le principe actif de l'Acupan®. Il s'agit d'un antalgique central non opioïde, de la famille des benzoxazocines (Klohs *et al.* (1972) *Arzneimittelforschung* **22:**132-3). Parmi ses avantages figure notamment l'absence d'effets dépresseurs respiratoires. Son mode d'action est encore mal connu mais semble faire appel à une inhibition de la recapture des monoamines, ce qui le distingue du paracétamol et des anti-inflammatoires non stéroïdiens (AINS). A l'heure actuelle, le néfopam est principalement utilisé pour le traitement de la douleur post-opératoire. En France, on le trouve ainsi administré chez environ 20% des patients ayant subi une intervention chirurgicale (Fletcher et al. (2008) Pain 137:441-51).

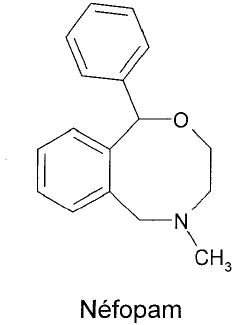

### Résumé de l'invention

La présente invention découle de la découverte inattendue, par les inventeurs, d'un effet analgésique synergique entre le néfopam et le célécoxib dans un modèle animal de douleur aiguë.

Cet effet analgésique synergique est particulièrement avantageux en ce qu'il permet de bénéficier d'une analgésie satisfaisante, sans atteinte gastrointestinale prévisible, et, du fait de la diminution de la dose à administrer du COXIB pour une même efficacité analgésique, une réduction du risque cardiovasculaire est anticipée.

Ainsi, la présente invention concerne une composition pharmaceutique comprenant à titre de substances actives :
a) au moins un composé de formule générale (I) suivante : 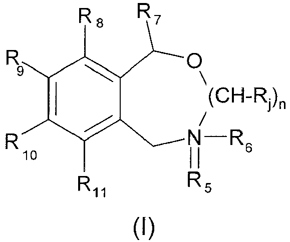 dans laquelle :
   - R₅ représente O ou aucun groupement ;
   - R₆ représente H ou un groupement alkyle contenant de 1 à 6 atomes de carbone ;
   - n représente un nombre entier de 2 à 4 ;
   - j représente un nombre entier variant de 1 à n ;
   - Rⱼ, identique ou différent pour chaque carbone substitué, représente H ou un groupement alkyle contenant de 1 à 6 atomes de carbone ;
   - R₇ représente un groupement phényle éventuellement substitué par un ou plusieurs groupements, identiques ou différents, choisi parmi la liste comprenant ou consistant en H, un groupement alkyle contenant de 1 à 6 atomes de carbone, un groupement alkoxy contenant de 1 à 6 atomes de carbones, un groupement trifluorométhyle, ou un atome d'halogène, de préférence I, Br, CI ou F ;
   - R₈, R₉, R₁₀, R₁₁ identiques ou différents représentent H, un groupement alkyle contenant de 1 à 6 atomes de carbone, un groupement alkoxy contenant de 1 à 6 atomes de carbone, un groupement trifluorométhyle, ou un atome d'halogène, de préférence I, Br, CI ou F ;
   ou un sel pharmaceutiquement acceptable de ce composé ; et
b) au moins un inhibiteur sélectif de cyclooxygénase de type 2 (COX-2) représenté par la formule (V) ci-bas ou un sel pharmaceutiquement acceptable de cet inhibiteur ;
éventuellement associés à un ou plusieurs excipients pharmaceutiquement acceptables,
notamment pour induire une analgésie ou pour son utilisation dans la prévention ou le traitement de la douleur.

La présente invention concerne également un composé de formule générale (I) telle que définie ci-dessus, ou un sel pharmaceutiquement acceptable de celui-ci, en combinaison avec un inhibiteur sélectif de COX-2 représenté par la formule (V) ci-bas tel que défini ci-dessus, ou un sel pharmaceutiquement acceptable de celui-ci, pour leur utilisation à titre de médicament, en particulier pour induire une analgésie ou pour prévenir ou traiter la douleur.

La présente invention concerne également l'utilisation d'un composé de formule générale (I) telle que définie ci-dessus, ou d'un sel pharmaceutiquement acceptable de celui-ci, en combinaison avec un inhibiteur sélectif de COX-2 représenté par la formule (V) ci-bas tel que défini ci-dessus, ou d'un sel pharmaceutiquement acceptable de celui-ci, pour la préparation d'un médicament analgésique ou destiné à la prévention ou au traitement de la douleur.

La présente invention concerne également une méthode d'induction d'une analgésie ou de prévention ou de traitement de la douleur chez un individu, dans laquelle on administre à l'individu une quantité prophylactiquement ou thérapeutiquement efficace d'au moins un composé de formule générale (I) telle que définie ci-dessus, ou d'un sel pharmaceutiquement acceptable de celui-ci, et une quantité prophylactiquement ou thérapeutiquement efficace d'au moins un inhibiteur sélectif de COX-2 représenté par la formule (V) ci-bas tel que défini ci-dessus, ou d'un sel pharmaceutiquement acceptable de celui-ci.

La présente invention concerne également des produits contenant :
- au moins un composé de formule générale (I) telle que définie ci-dessus, ou un sel pharmaceutiquement acceptable de celui-ci, et
- au moins un inhibiteur sélectif de COX-2 représenté par la formule (V) ci-bas tel que défini ci-dessus, ou un sel pharmaceutiquement acceptable de celui-ci,
comme produit de combinaison pour une utilisation ensemble ou de manière séparée pour induire une analgésie ou pour prévenir ou traiter la douleur.

Dans un mode de réalisation préféré de la composition pharmaceutique, des composés, de l'utilisation, de la méthode et des produits définis ci-dessus, au moins un composé analgésique ou antalgique additionnel, différent des composés de formules générales (I) et de l'inhibiteur sélectif de COX-2 représenté par la formule (V) ci-bas tels que définis ci-dessus, ou de leurs sels pharmaceutiquement acceptables, est ajouté en combinaison avec les composés de formules générales (I) et l'inhibiteur sélectif de COX-2 tels que définis ci-dessus.

Toutefois, selon l'invention , aucun composé de formule générale (VI) suivante, ou de sel pharmaceutiquement acceptable de celui-ci, n'est compris dans la composition pharmaceutique ou les produits, ou n'est en association ou en combinaison avec les composés de formules générales (I) et l'inhibiteur sélectif de COX-2 représenté par la formule (V) tels que définis ci-dessus : 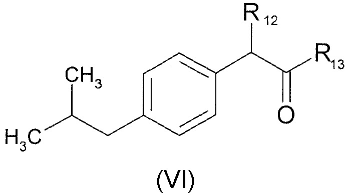
- R₁₂ représentant H ou un groupement alkyle contenant de 1 à 6 atomes de carbone ;
- R₁₃ représentant un groupement OR₁₄ ou NR₁₅R₁₆ ;
- R₁₄ représentant H, un groupement alkyle contenant de 1 à 6 atomes de carbone, un groupement aryle contenant de 6 à 10 atomes de carbone, ou un groupement aralkyle ou alkaryle contenant de 7 à 20 atomes de carbone ;
- R₁₅ et R₁₆ identiques ou différents représentant H, OH, un groupement alkyle contenant de 1 à 6 atomes de carbone, un groupement aryle contenant de 6 à 10 atomes de carbone, ou un groupement aralkyle ou alkaryle contenant de 7 à 20 atomes de carbone.

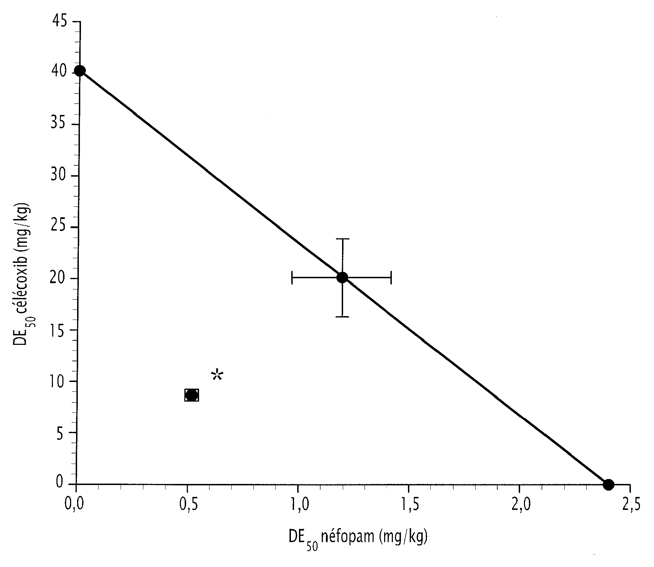

### Description de la figure 1

La figure 1 représente l'isobologramme de l'association néfopam-célécoxib dans le test des crampes induites par l'acide acétique chez la souris. Le symbole étoile (*) indique que le point représentatif de la DE50 de l'association néfopam-célécoxib mesurée expérimentalement est situé sous la droite d'additivité de manière statistiquement significative.

### Description détaillée de l'invention

### Prévention ou traitement de la douleur

L'expression « traiter la douleur » signifie diminuer ou abolir une douleur ou la sensibilité à cette douleur. L'expression « prévenir la douleur » signifie que les composés de formules générales (I) et l'inhibiteur sélectif de COX-2 tels que définis ci-dessus, ou des sels pharmaceutiquement acceptables de ceux-ci, sont administrés à un individu avant que cet individu ne perçoive la douleur à traiter.

L'expression « induire une analgésie » signifie aussi bien diminuer ou abolir une douleur que la sensibilité à cette douleur. On considère ici que cette expression est équivalente à « une utilisation en tant qu'analgésique ou antalgique ».

L'invention vise à la prévention ou au traitement de toute douleur quelle que soit son origine.

On préfère, toutefois, que la douleur prévenue ou traitée selon l'invention soit une douleur aiguë. L'expression « douleur aiguë » est bien connue de l'homme du métier. Elle s'oppose à la notion de « douleur chronique ». D'une manière générale on considère qu'une douleur aiguë est une douleur dont la durée est inférieure à 3 mois.

On préfère également que la douleur prévenue ou traitée selon l'invention, soit une douleur faisant partie des indications habituelles des composés de formule (I) ou de leurs sels pharmaceutiquement acceptables selon l'invention, notamment du néfopam, ou des inhibiteurs sélectifs de COX-2 représenté par la formule (V) ou de leurs sels pharmaceutiquement acceptables selon l'invention, notamment du célécoxib.

Ainsi, de préférence également, la douleur prévenue ou traitée selon l'invention est une douleur aiguë post-opératoire, une douleur rhumatologique, une douleur liée à une dysménorrhée, et/ou une douleur liée à une polypose adénomateuse familiale. De manière particulièrement préférée, la douleur prévenue ou traitée selon l'invention est une douleur aiguë post-opératoire et/ou une douleur rhumatologique.

On désigne par « douleur aiguë post-opératoire » une douleur qui trouve son origine dans une intervention chirurgicale ; en particulier, une intervention chirurgicale lors de laquelle est réalisée une incision, dont la cicatrisation crée une douleur de type inflammatoire avec une éventuelle participation hyperalgésique, pendant une durée moyenne de 5 à 7 jours (Chauvin et Clergue (1998) Ann. Fr. Anesth. Réanim. 17:444).

La « douleur rhumatologique » selon l'invention est de préférence une douleur liée à l'arthrose, à l'ostéoporose, à l'arthrite, notamment infectieuse ou inflammatoire, en particulier l'ostéoarthrite, à la goutte, à une fibromyalgie, aux rhumatismes inflammatoires, comme la polyarthrite rhumatoïde, notamment juvénile, ou la spondylarthrite ankylosante.

De préférence, l'intensité de la douleur prévenue ou traitée selon l'invention est au moins modérée, plus préférablement au moins sévère (également nommée forte). Les notions de « douleur modérée » ou de « douleur sévère » sont bien connues de l'homme du métier. A titre d'exemple, on considère généralement qu'une douleur modérée correspond à un index de 4 à 6 et une douleur sévère à un index de 7 à 9 sur une échelle numérique de douleur graduée de 0 à 10. Sur cette même échelle l'index 0 correspond à une absence de douleur, l'index 1 à 3 à une douleur légère, et l'index 10 à la douleur maximale imaginable.

### Composé de formule générale (I)

De préférence, le composé de formule générale (I) définie ci-dessus est représenté par l'une des formules (II), (III) et (IV) suivantes :

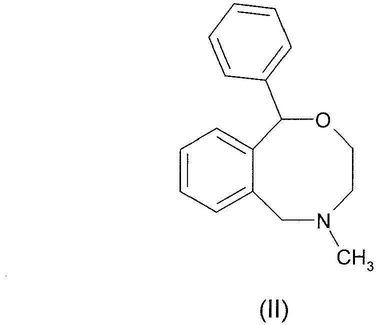

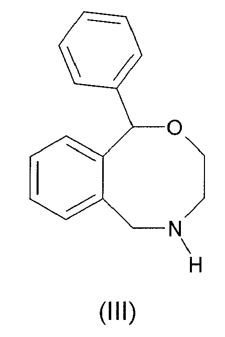

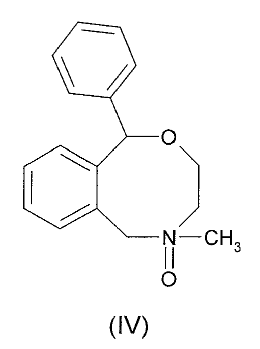

La formule (II) représente le néfopam, et les formules (III) et (IV) représentent respectivement deux métabolites du néfopam à savoir le desméthyle néfopam et le *N*-oxyde néfopam.

De manière particulièrement préférée, le composé de formule générale (I) telle que définie ci-dessus est représenté par la formule (II) ci-dessus et correspond au néfopam.

Comme on l'entend ici, la formule générale (I) définie ci-dessus représente également les stéréoisomères et mélanges de stéréoisomères, notamment le mélange racémique, des composés de formule (I).

Les sels pharmaceutiquement acceptables des composés de formule générale (I) définie ci-dessus apparaîtront clairement à l'homme du métier. En particulier, les sels de chlorhydrate des composés de formule générale (I) telle que définie ci-dessus sont préférés.

Le chlorhydrate de néfopam est le composé de formule générale (I) telle que définie ci-dessus le plus préféré pour la mise en oeuvre de l'invention.

### Inhibiteur sélectif de COX-2

Comme on l'entend ici un inhibiteur sélectif de COX-2 est tel qu'il inhibe la COX-2 de manière plus importante que la COX-1, l'inhibition de la COX-2 et de la COX-1 étant mesurée dans les mêmes conditions. On considère en particulier qu'un inhibiteur est sélectif de la COX-2 lorsque l'index de sélectivité de l'inhibiteur, c'est-à-dire que le rapport IC50_{COX-2} / IC50_{COX-1} de l'inhibiteur, en particulier mesurés dans du sang total, est inférieur à 1, de préférence inférieur à 0,5 et plus préférablement inférieur à 0,2. L'expression « IC50_{COX} », bien connue de l'homme du métier, désigne la concentration en inhibiteur permettant d'inhiber 50% de l'activité maximale de la COX en question.

De préférence, l'IC50 des inhibiteurs sélectifs de COX-2 selon l'invention vis-à-vis de la COX-2, en particulier mesuré dans le sang total, est inférieure à 5 µM, de préférence inférieure à 2 µM et plus préférablement inférieure à 1 µM.

La détermination de l'inhibition de COX-1 et COX-2, notamment dans le sang total, peut être aisément mise en oeuvre par l'homme du métier. En particulier, la détermination de l'inhibition de COX-1 et COX-2 dans le sang total peut être mise en oeuvre comme cela est indiqué par Cryer & Feldman (1998) Am. J. Med. 104:413-421, notamment pages 414 et 415, paragraphes *COX-1 whole blood assay* et *COX-2 whole blood assay.*

De préférence également, l'inhibiteur sélectif de COX-2 selon l'invention appartient à la classe médicamenteuse (ou thérapeutique) des COXIBs.

L'inhibiteur sélectif de COX-2 selon l'invention est représenté par la formule générale (V) suivante : 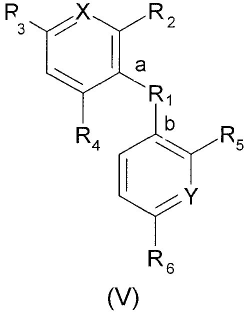 dans laquelle :
- R₁ est sélectionné dans le groupe constitué de 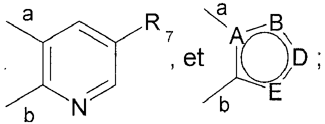
- a et b permettent de déterminer l'orientation de R₁ ;
- R₂ représente H ou un halogène, de préférence I, Br, CI ou F, et plus préférablement CI ;
- R₃ représente H ou -SO₂R₈, où R₈ représente -CH₃, -NH₂ ou -NH-CO-CH₂-CH₃ ;
- R₄ représente H ou un halogène, de préférence I, Br, CI ou F, et plus préférablement F ;
- R₅ représente H ou CH₂-COOH ;
- R₆ représente H ou CH₃ ;
- R₇ représente H ou un halogène, de préférence I, Br, CI ou F, et plus préférablement Cl ;
- X et Y représentent indépendamment N ou CH ;
- A représente N ou C ;
- B représente N, CH₂ ou C-CH₃ ;
- D représente, O, ou C-CF₃ ;
- E représente N, CH ou C=O.

De manière davantage préférée, l'inhibiteur sélectif de COX-2 selon l'invention est représenté par la formule générale (V) définie ci-dessus, sous réserve qu'il soit différent du diclofénac.

De manière encore plus préférée, l'inhibiteur sélectif de COX-2 selon l'invention est représenté par la formule générale (V) définie ci-dessus, dans laquelle R₁ est sélectionné dans le groupe constitué de :

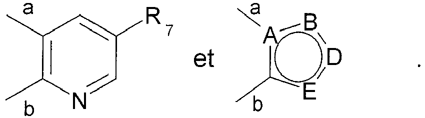

De manière particulièrement préférée, l'inhibiteur sélectif de COX-2 selon l'invention est choisi dans le groupe constitué du célécoxib, du parécoxib, du valdécoxib, du rofécoxib, et de l'étoricoxib ; et de manière plus préférée encore l'inhibiteur sélectif de COX-2 selon l'invention est le célécoxib.

Les formules des inhibiteurs sélectifs de COX-2 préférés selon l'invention sont représentées ci-dessous :

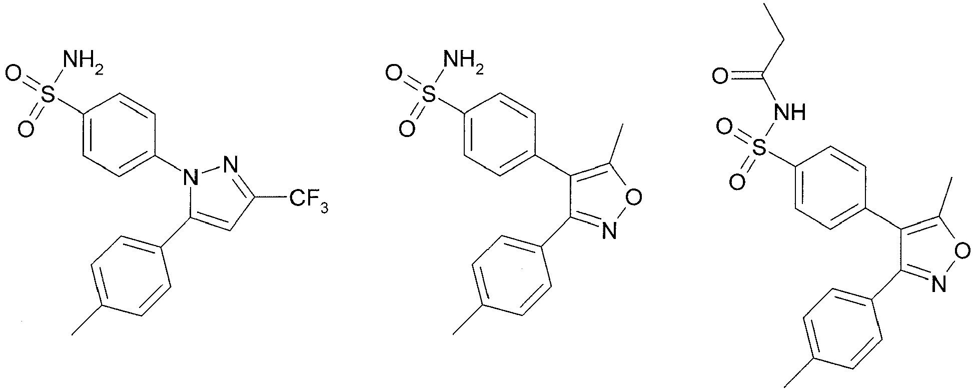

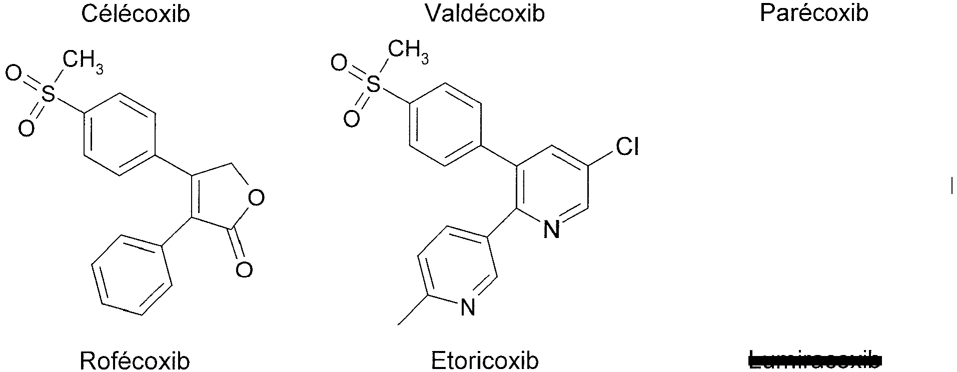

### Administration

Comme on l'entend ici l'expression « en combinaison » ou « produit de combinaison » signifie que le composé de formule générale (I) telle que définie ci-dessus, ou un sel pharmaceutiquement acceptable de celui-ci, et l'inhibiteur sélectif de COX-2 représenté par la formule (V) tel que défini ci-dessus, ou un sel pharmaceutiquement acceptable de celui-ci, peuvent être associés au sein d'une même composition pharmaceutique, et donc être administrés ensemble, ou bien être administrés de manière séparée, c'est-à-dire selon des voies d'administration distinctes et/ou des régimes d'administration distincts, sous réserve que lorsqu'ils sont administrés de manière séparée les périodes d'activité analgésique respectives du composé de formule générale (I) et de l'inhibiteur sélectif de COX-2 représenté par la formule (V) se recouvrent en totalité ou en partie, notamment de manière que les composés puissent coopérer pour exercer un effet analgésique synergique.

Ainsi, lorsque les composés sont administrés de manière séparée, le composé de formule générale (I) telle que définie ci-dessus, ou son sel pharmaceutiquement acceptable, sera de préférence administré dans les 24 heures, plus préférablement dans les 2 heures, et encore plus préférablement dans l'heure, suivant l'administration de l'inhibiteur sélectif de COX-2 représenté par la formule (V) tel que défini ci-dessus, ou de son sel pharmaceutiquement acceptable, et son administration sera éventuellement poursuivie les jours suivants. Réciproquement, l'inhibiteur sélectif de COX-2 r représenté par la formule (V) tel que défini ci-dessus, ou son sel pharmaceutiquement acceptable, sera de préférence administré dans les 24 heures, plus préférablement dans les 2 heures, et encore plus préférablement dans l'heure, suivant l'administration du composé de formule générale (I) telle que définie ci-dessus, ou de son sel pharmaceutiquement acceptable, et son administration sera éventuellement poursuivie les jours suivants. Dans un autre mode de réalisation préférée de l'invention, lorsque les composés selon l'invention sont administrés de manière séparée, ils sont administrés essentiellement simultanément.

De préférence, le composé de formule générale (I) telle que définie ci-dessus, ou son sel pharmaceutiquement acceptable, est administré ou se trouve sous une forme convenant à une administration par voie orale, intraveineuse ou intramusculaire.

De préférence, l'inhibiteur sélectif de COX-2 représenté par la formule (V) tel que défini ci-dessus, ou son sel pharmaceutiquement acceptable, est administré ou se trouve sous une forme convenant à une administration par voie orale, injectable ou locale.

De préférence, lorsque le composé de formule générale (I) telle que définie ci-dessus, ou son sel pharmaceutiquement acceptable, et l'inhibiteur spécifique de COX-2 représenté par la formule (V) tel que défini ci-dessus, ou son sel pharmaceutiquement acceptable, sont associés au sein d'une même composition pharmaceutique, celle-ci est administrée ou se trouve sous une forme convenant à une administration par voie orale, injectable ou locale.

De préférence, le composé de formule (I) telle que définie ci-dessus, ou son sel pharmaceutiquement acceptable, notamment le chlorhydrate de néfopam, se trouve contenu dans les compositions pharmaceutiques ou les produits définis ci-dessus, ou est administré, à une dose unitaire de 1 mg à 120 mg, plus préférablement à une dose unitaire de 20 mg.

De préférence, l'inhibiteur sélectif de COX-2, représenté par la formule (V) ou son sel pharmaceutiquement acceptable, notamment le célécoxib, se trouve contenu dans les compositions pharmaceutiques ou les produits définis ci-dessus, ou est administré, à une dose unitaire de 10 mg à 1000 mg, plus préférablement à une dose unitaire de 50 mg à 500 mg.

### Composé analgésique ou antalgique additionnel

N'importe quel composé analgésique ou antalgique peut convenir à titre de composé analgésique ou antalgique additionnel selon l'invention, il est préféré, toutefois, qu'il s'agisse :
- d'un morphinique, tel que la morphine, le fentanyl, le remifentanil, l'alfentanil, le sufentanyl, la nalbuphine, la pentazocine, la codéine, l'hydrocodéine, la dihydrocodéine, le dextropropoxyphène, le tramadol, la buprénorphine, l'hydromorphone, l'oxycodone, ou la péthidine ;
- d'un anti-inflammatoire non stéroïdien (AINS), tel que le kétoprofène, l'acide acétylsalicylique, l'acide méfénamique, le fénoprofène, l'acéclofénac, l'acide tiaprofénique, l'alminoprofène, le diclofénac, l'étodolac, le flurbiprofène, la nabumétone, le naproxène, le méloxicam, le piroxicam, le ténoxicam, l'indométacine, le sulindac, la floctafénine, la phénylbutazone, ou le nimésulide ;
- du paracétamol, du ziconitide, ou de la caféine.

### Composé de formule générale (VI)

Le composé de formule générale (VI) définie ci-dessus est préférentiellement représenté par la formule (VII) suivante : 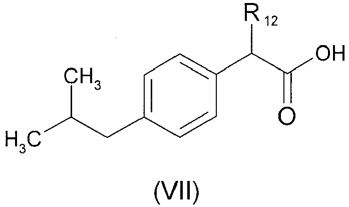 dans laquelle R₁₂ est tel que défini ci-dessus.

De préférence également, le composé de formule générale (VI) définie ci-dessus est représenté par les formules (VIII), (IX) et (X) suivantes :

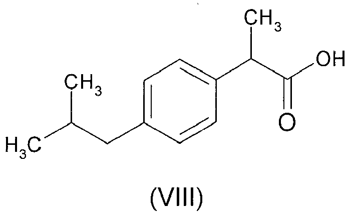

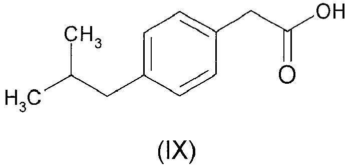

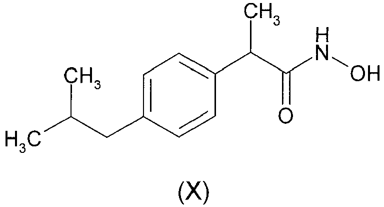

Les formules (VIII), (IX) et (X) ci-dessus représentent respectivement l'ibuprofène, l'ibufénac et l'ibuproxam.

De manière davantage préférée le composé de formule générale (VI) définie ci-dessus est représenté par la formule (VIII) ci-dessus et de manière particulièrement préférée par la formule (XI) suivante :

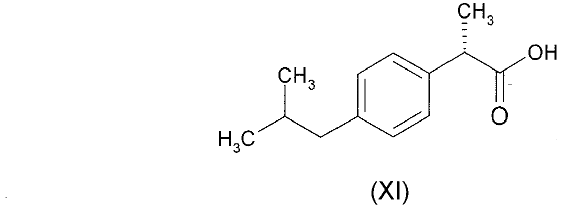

La formule (XI) représente la forme S de l'ibuprofène qui porte l'essentiel des propriétés analgésiques de l'ibuprofène.

Comme on l'entend ici, les formules (VI), (VII), (VIII) et (X) définies ci-dessus représentent également les stéréoisomères et mélanges de stéréoisomères, notamment le mélange racémique, des composés de formules (VI), (VII), (VIII) et (X).

Les sels pharmaceutiquement acceptables des composés de formule générale (VI) définie ci-dessus apparaîtront clairement à l'homme du métier. En particulier, les sels de lysine, notamment de L-lysine, des composés de formule générale (V) telle que définie ci-dessus sont préférés, tels que le sel de lysine de l'ibuprofène ou le sel monohydraté de L-lysine de la forme S de l'ibuprofène.

Par ailleurs, comme cela apparaîtra clairement à l'homme du métier, il est aisé de synthétiser des prodrogues des composés de formules (VII), (VIII) et (IX) définies ci-dessus, c'est-à-dire des composés qui sont rapidement transformés *in vivo* pour donner le composé de formules (VII), (VIII) et (IX) définies ci-dessus, par exemple par hydrolyse dans le sang. Aussi, au-delà des prodrogues des composés de formules (VII), (VIII) et (IX) définies ci-dessus qui sont représentées par les composés de formule (VI) pour lesquelles R₁₃ est différent de OH, tel que l'ibuproxam, on vise de préférence ici à exclure la mise en oeuvre de l'ensemble des prodrogues des composés de formule (VI) définie ci-dessus.

### Exemple

L'effet analgésique d'une association néfopam - célécoxib a été étudié dans le modèle de crampes abdominales induites chez la souris par l'administration intrapéritonéale d'acide acétique. Ce modèle de douleur induite par une substance chimique correspond à une douleur inflammatoire, viscérale et aiguë.

### A. Matériel et méthodes

### 1. Animaux

Des souris mâles CD1 (élevage C. River) de poids compris entre 25 et 30 grammes sont utilisées après une acclimatation d'au moins 7 jours dans l'animalerie (t° = 22 ± 2 ; hygrométrie = 50 ± 20 % ; nourriture SAFE "A04"; cycle nycthéméral : 12 h/12 h (lumière : 7 h/19 h - obscurité : 19 h/7 h)).

### 2. Protocole

Le jour de l'expérimentation, les souris non à jeun sont pesées, marquées et réparties au hasard par lot de 10. La solution d'acide acétique (Sigma) est préparée à 0,6 % soit 60 mg d'acide acétique dans 10 ml de NaCl à 0,9 %.

A t = 0, la souris reçoit le célécoxib par voie orale. A t = 30 min, la souris reçoit le néfopam ou son liquide véhicule par voie sous-cutanée. A t = 60 min, l'acide acétique est injecté par voie intrapéritonéale (0,1 ml/10 g).

On compte le nombre de crampes abdominales de 5 à 20 minutes après l'injection d'acide acétique. On considère comme positives les crampes abdominales franches caractérisées par l'étirement des pattes postérieures et/ou le creusement des flancs avec une torsion.

### 3. Produits

Le chlorhydrate de néfopam (Biocodex, lot 38) (ci-après désigné simplement néfopam) est solubilisé dans l'eau distillée ou du NaCl à 0,9 %. Le célécoxib (Célébrex® gélules) est mis en suspension dans du Tween 80 à 1 %.

### 4. Analyse statistique

Le test utilisé est une analyse de variance à partir de 3 groupes et un test t de Student pour 2 groupes. Ensuite, on détermine le ou les groupes traités qui diffèrent du groupe témoin. Le calcul des doses efficaces à 50 % d'effet antinociceptif (DE50) se fait à l'aide du programme PharmToolsPro (version 1.1.27, McCary Group Inc.) selon la méthode de Tallarida (2000) Drugs synergism and dose-effect data analysis CRC Press). Au moins 10 souris sont utilisées pour chaque dose, et au moins 3 doses sont employées pour déterminer la courbe dose-réponse. La dose qui produit 50 % d'effet antinociceptif (diminution de 50 % du nombre de crampes) est calculée par une analyse de régression linéaire standard de la courbe dose-réponse.

L'interaction est évaluée par une analyse isobolographique de la co-administration d'une combinaison de doses à rapport fixe selon Tallarida (2000) op. cit.*,* Tallarida et al. (1989) Life Sci. 45:947-961 et Tallarida et al. (1997) Life Sci. 61:PL417-PL425. L'isobologramme est construit en joignant la DE50 du célécoxib avec celle du néfopam pour obtenir la ligne d'additivité. La DE50 de l'association est déterminée par analyse de régression linéaire de la courbe dose-réponse, et elle est comparée par un test *t* à une DE50 additive théorique obtenue à l'aide du logiciel PharmToolsPro.

### B. Résultats

### 1. Néfopam seul

L'administration sous-cutanée de néfopam entraîne une inhibition dose-dépendante du nombre de crampes induites par l'acide acétique chez la souris (**Tableau 1**). La DE50 mesurée du néfopam est de 2,395 ± 0,215 mg/kg.

### 2. Célécoxib seul

Le célécoxib administré par voie orale diminue de façon dose-dépendante le nombre de crampes induites par l'acide acétique chez la souris, avec une DE50 de 40,172 ± 8,060 mg/kg **(Tableau 1).**

Ce résultat est similaire à celui obtenu par Lu et al. (2005) Acta Pharmacol. Sin. 26:1505-1511 dans le même modèle (94 mg/kg).

**Tableau 1 : effets propres du néfopam et du célécoxib administrés seuls**

| **Produits (mg/kg)** | **n** | **Nombre de crampes (moyennes±esm)** | **% variation** | **ANOVA** |
|---|---|---|---|---|
| **Néfopam** | | | | |
| 0 | 18 | 37,1 ± 2,8 | | |
| 0,3 | 9 | 33,6 ± 3,8 | -9 | ns |
| 1,0 | 10 | 28,0 ± 6,3 | -25 | ns |
| 3,0 | 18 | 16,7 ± 2,0 | -55 | p < 0,05 |
| 10,0 | 10 | 7,1 ± 1,7 | -81 | p < 0,05 |
| 20,0 | 10 | 0,6 ± 0,3 | -98 | p<0,05 |
| | | **DE50 (mg/kg) = 2,395 ± 0,215** | | |

| **Célécoxib** | | | | |
|---|---|---|---|---|
| 0 | 18 | 31,0 ± 2,9 | | |
| 10 | 10 | 28,2 ± 2,9 | -9 | ns |
| 20 | 10 | 18,1 ± 3,2 | -42 | ns |
| 50 | 10 | 18,9 ± 2,2 | -39 | ns |
| 75 | 10 | 8,6 ± 2,1 | -72 | p < 0,05 |
| 150 | 10 | 3,2 ± 1,0 | -90 | p < 0,05 |
| | | **DE50 (mg/kg) = 40,172 ± 8,060** | | |

| | | | | |
|---|---|---|---|---|
| (p < 0,05 : test statistique ANOVA suivi de Bonferoni ou Dunn) | | | | |

### 3. Association néfopam - célécoxib

Dans un premier temps, on détermine, à l'aide du logiciel PharmToolsPro, la proportion fixe de chaque produit pour un niveau d'efficacité de 50 % et la DE50 théorique qui se situe sur la droite d'additivité, selon Tallarida (2000) *op. ci*t*.* On obtient ainsi une proportion de 0,056 pour le néfopam et de 0,944 pour le célécoxib et une DE50 théorique de 21,330 ± 4,041 mg/kg.

Dans un deuxième temps, des compositions ayant une proportion de 5,6 % de néfopam et de 94,4 % de célécoxib sont étudiées dans le modèle animal afin d'obtenir une DE50 expérimentale qui sera comparée à la DE50 théorique de la droite d'additivité. Le **Tableau 2** montre les résultats expérimentaux obtenus. La 19 DE50 expérimentale est de 9,193 ± 0,542 mg/kg (correspondant à 0,515 ± 0,030 mg/kg de néfopam et 8,678 ± 0,512 mg/kg de célécoxib).

Enfin, dans un dernier temps, la DE50 expérimentale est placée sur l'isobologramme obtenu à partir des données du **Tableau 1 (****Figure 1****).** On observe que la DE50 expérimentale de l'association néfopam - célécoxib est située en dessous de la droite d'additivité, où se situe la DE50 théorique correspondant à une additivité simple. L'interaction entre le néfopam et l'ibuprofène se situe donc dans la zone de supra-additivité indiquant une relation synergique entre les deux composés. Par ailleurs, l'analyse statistique (test t de Student) donne un *t_{expérimental}* de 4,236 qui est supérieur au *t_{théorique}* de 2,329, par conséquent la différence entre la DE50 expérimentale et la DE50 théorique est significative.

**Tableau 2 : effet de la co-administration du néfopam et du célécoxib**

| **Néfopam (mg/kg)** | **Célécoxib (mg/kg)** | **n** | **Nombre de crampes (moyennes esm)** | **% variation** | **ANOVA** |
|---|---|---|---|---|---|
| 0 | 0 | 12 | 30,2 ± 2,8 | | |
| 0,075 | 1,25 | 10 | 26,8 ± 5,4 | -11 | ns |
| 0,15 | 2,5 | 10 | 24,2 ± 2,9 | -20 | ns |
| 0,30 | 5 | 10 | 19,2 ± 2,4 | -36 | p < 0,05 |
| 0,60 | 10 | 9 | 13,1 ± 2,6 | -57 | p < 0,05 |
| 1,20 | 20 | 12 | 9,1 ± 2,3 | -70 | p < 0,05 |
| 2,40 | 40 | 12 | 5,2 ± 1,1 | -83 | p < 0,05 |
| 4,80 | 80 | 12 | 1,8 ± 0,6 | -94 | p < 0,05 |
| | | | **DE50 (mg/kg)= 9,193 ± 0,542** | | |

| | | | | | |
|---|---|---|---|---|---|
| (p < 0,05 : test statistique ANOVA suivi de Bonferoni ou Dunn) | | | | | |

## Revendications

1. Composition pharmaceutique comprenant à titre de substances actives :
a) au moins un composé de formule générale (I) suivante : 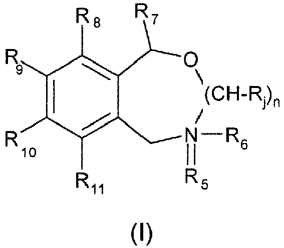 dans laquelle :
- R₅ représente O ou aucun groupement ;
- R₆ représente H ou un groupement alkyle contenant de 1 à 6 atomes de carbone ;
- n représente un nombre entier de 2 à 4 ;
- j représente un nombre entier variant de 1 à n ;
- Rⱼ, identique ou différent pour chaque carbone substitué, représente H ou un groupement alkyle contenant de 1 à 6 atomes de carbone ;
- R₇ représente un groupement phényle éventuellement substitué par un ou plusieurs groupements, identiques ou différents, choisi parmi la liste comprenant ou consistant en H, un groupement alkyle contenant de 1 à 6 atomes de carbone, un groupement alkoxy contenant de 1 à 6 atomes de carbones, un groupement trifluorométhyle, ou un atome d'halogène ;
- R₈, R₉, R₁₀, R₁₁ identiques ou différents représentent H, un groupement alkyle contenant de 1 à 6 atomes de carbone, un groupement alkoxy contenant de 1 à 6 atomes de carbone, un groupement trifluorométhyle, ou un atome d'halogène ;
ou un sel pharmaceutiquement acceptable de ce composé ; et
b) au moins un inhibiteur sélectif de la cyclooxygénase de type 2 (COX-2) représenté par la formule (V) suivante : 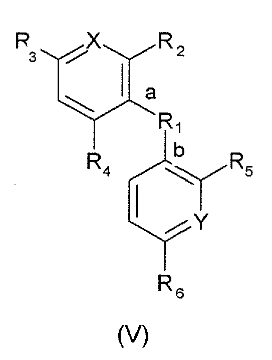 dans laquelle :
- R₁ est sélectionné dans le groupe constitué de 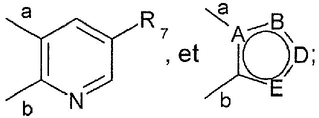
- a et b permettent de déterminer l'orientation de R₁ ;
- R₂ représente H ou un halogène, de préférence I, Br, Cl ou F, et plus préférablement Cl ;
- R₃ représente H ou -SO₂R₈, où R₈ représente -CH₃, -NH₂ ou -NH-CO-CH₂-CH₃ ;
- R₄ représente H ou un halogène, de préférence I, Br, Cl ou F, et plus préférablement F ;
- R₅ représente H ou CH₂-COOH ;
- R₆ représente H ou CH₃ ;
- R₇ représente H ou un halogène, de préférence I, Br, Cl ou F, et plus préférablement Cl ;
- X et Y représentent indépendamment N ou CH ;
- A représente N ou C ;
- B représente N, CH₂ ou C-CH₃ ;
- D représente, O, ou C-CF₃ ;
- E représente N, CH ou C=O ;
ou un sel pharmaceutiquement acceptable de cet inhibiteur ;
éventuellement associés à un ou plusieurs excipients pharmaceutiquement acceptables,
la composition pharmaceutique ne comprenant pas de composé de formule générale (VI) suivante, ou de sel pharmaceutiquement acceptable de celui-ci : 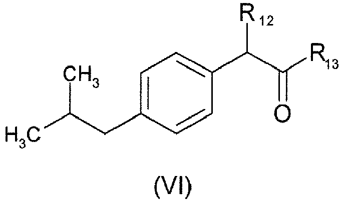
- R₁₂ représentant H ou un groupement alkyle contenant de 1 à 6 atomes de carbone ;
- R₁₃ représentant un groupement OR₁₄ ou NR₁₅R₁₆ ;
- R₁₄ représentant H, un groupement alkyle contenant de 1 à 6 atomes de carbone, un groupement aryle contenant de 6 à 10 atomes de carbone, ou un groupement aralkyle ou alkaryle contenant de 7 à 20 atomes de carbone ;
- R₁₅ et R₁₆ identiques ou différents représentant H, OH, un groupement alkyle contenant de 1 à 6 atomes de carbone, un groupement aryle contenant de 6 à 10 atomes de carbone, ou un groupement aralkyle ou alkaryle contenant de 7 à 20 atomes de carbone.

2. Composition pharmaceutique selon la revendication 1, dans laquelle le composé de formule (I) est représenté par une des formules (II), (III) et (IV) suivantes :

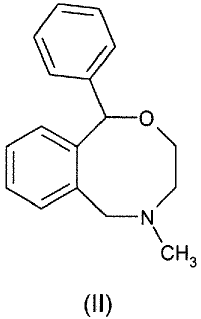

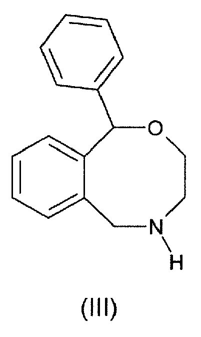

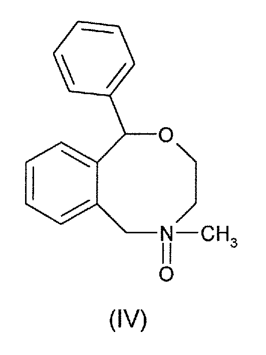

3. Composition pharmaceutique selon la revendication 1 ou 2, dans laquelle l'inhibiteur sélectif de COX-2 est sélectionné dans le groupe constitué du célécoxib, du parécoxib, du valdécoxib, du rofécoxib, et de l'étoricoxib.

4. Composition pharmaceutique selon l'une des revendications 1 à 3, comprenant :
a) du chlorhydrate de néfopam, et
b) le célécoxib.

5. Composition pharmaceutique selon l'une des revendications 1 à 4, comprenant une dose unitaire de 1 mg à 120 mg du composé de formule (I) ou d'un sel pharmaceutiquement acceptable de celui-ci.

6. Composition pharmaceutique selon l'une des revendications 1 à 5, comprenant une dose unitaire de 10 mg à 1000 mg de l'inhibiteur sélectif de COX-2 ou d'un sel pharmaceutiquement acceptable de celui-ci.

7. Composition pharmaceutique selon l'une des revendications 1 à 6, convenant pour une administration par voie orale, injectable ou locale.

8. Composition pharmaceutique selon l'une des revendications 1 à 7, pour induire une analgésie ou pour son utilisation dans la prévention ou le traitement de la douleur.

9. Composé de formule générale (I), ou sel pharmaceutiquement acceptable de celui-ci, tel que défini dans l'une des revendications 1, 2 et 4, en combinaison avec
un inhibiteur sélectif de COX-2, ou un sel pharmaceutiquement acceptable de celui-ci, tel que défini dans l'une des revendications 1, 3 et 4,
pour leur utilisation à titre de médicament,
le composé de formule générale (I) et l'inhibiteur sélectif de COX-2, ou leur sels pharmaceutiquement acceptables, n'étant pas en combinaison avec un composé de formule (VI) telle que définie dans la revendication 1, ou un sel pharmaceutiquement acceptable de celui-ci.

10. Composés ou sels pharmaceutiquement acceptables de ceux-ci selon la revendication 9, dans lesquels le composé de formule (I), ou un sel pharmaceutiquement acceptable de celui-ci, est administré à la dose unitaire de 1 mg à 120 mg.

11. Composés ou sels pharmaceutiquement acceptables de ceux-ci selon la revendication 9 ou 10, dans lesquels l'inhibiteur sélectif de COX-2, ou un sel pharmaceutiquement acceptable de celui-ci, est administré à la dose unitaire de 10 mg à 1000 mg.

12. Composés ou sels pharmaceutiquement acceptables de ceux-ci selon l'une des revendications 9 à 11, dans lesquels le composé de formule (I), ou un sel pharmaceutiquement acceptable de celui-ci, est administré par voie intraveineuse, orale, ou intramusculaire.

13. Composés ou sels pharmaceutiquement acceptables de ceux-ci selon l'une des revendications 9 à 12, dans lesquels l'inhibiteur sélectif de COX-2, ou un sel pharmaceutiquement acceptable de celui-ci, est administré par voie orale, injectable ou locale.

14. Composés ou sels pharmaceutiquement acceptables de ceux-ci selon l'une des revendications 9 à 13, pour induire une analgésie ou pour leur utilisation dans la prévention ou le traitement de la douleur.

15. Produits contenant :
- au moins un composé de formule générale (I), ou un sel pharmaceutiquement acceptable de celui-ci, tel que défini dans l'une des revendications 1, 2 et 4, et
- au moins un inhibiteur sélectif de COX-2, ou un sel pharmaceutiquement acceptable de celui-ci, tel que défini dans l'une des revendications 1, 3 et 4, comme produit de combinaison pour une utilisation ensemble ou de manière séparée pour induire une analgésie ou pour prévenir ou traiter la douleur,
les produits ne comprenant pas de composé de formule générale (VI) telle que définie dans la revendication 1, ou un sel pharmaceutiquement acceptable de celui-ci.

16. Produits selon la revendication 15, comprenant une dose unitaire de 1 mg à 120 mg du composé de formule (I), ou d'un sel pharmaceutiquement acceptable de celui-ci.

17. Produits selon la revendication 15 ou 16, comprenant une dose unitaire de 10 mg à 1000 mg de l'inhibiteur sélectif de COX-2, ou d'un sel pharmaceutiquement acceptable de celui-ci.

18. Produits selon l'une des revendications 15 à 17, dans lequel le composé de formule (I), ou un sel pharmaceutiquement acceptable de celui-ci, est sous une forme administrable par voie intraveineuse, orale, ou intramusculaire.

19. Produits selon l'une des revendications 15 à 18, dans lesquels l'inhibiteur sélectif de COX-2, ou un sel pharmaceutiquement acceptable de celui-ci, est sous une forme administrable par voie orale, injectable ou locale.

## Patentansprüche

1. Pharmazeutische Zubereitung, als aktive Substanz enthaltend:
a) mindestens eine Verbindung der folgenden allgemeinen Formel (I): 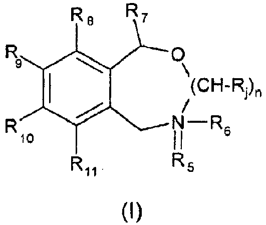 in der
- R₅ für O oder keine Gruppe steht,
- R₆ für H oder eine Alkyl-Gruppe mit 1 bis 6 Kohlenstoffatomen,
- n eine ganze Zahl von 2 bis 4 ist,
- j eine ganze Zahl von 1 bis n,
- Rⱼ, identisch oder bei jedem substituierten Kohlenstoffatom verschieden, für H oder eine Alkyl-Gruppe mit 1 bis 6 Kohlenstoffatomen steht,
- R₇ für eine Phenylgruppe, die gegebenenfalls mit einer oder mehreren, identischen oder verschiedenen Gruppen substituiert ist, die aus der Liste ausgewählt wurden, die aus H, einer Alkyl-Gruppe mit 1 bis 6 Kohlenstoffatomen, einer Alkoxy-Gruppe mit 1 bis 6 Kohlenstoffatomen, einer Trifluormethyl-Gruppe oder einem Halogenatom besteht,
- R₈, R₉, R₁₀, R₁₁, identisch oder verschieden, für H oder eine Alkyl-Gruppe mit 1 bis 6 Kohlenstoffatomen, eine Alkoxy-Gruppe mit 1 bis 6 Kohlenstoffatomen, eine Trifluormethyl-Gruppe oder ein Halogenatom,
oder ein pharmazeutisch annehmbares Salz dieser Verbindung, und
b) mindestens einen selektiven Inhibitor von Zyklooxygenase Typ 2 (COX-2), der der folgenden Formel (V) entspricht: 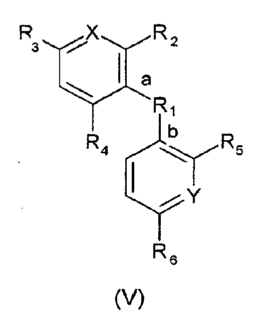 in der
- R₁ aus der Gruppe ausgewählt wurde, die aus 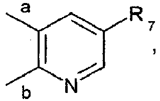
und 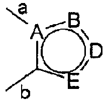 besteht,
- a und b die Orientierung von R₁ zu bestimmen erlauben,
- R₂ für H oder ein Halogen, vorzugsweise I, Br, Cl oder F, und besonders bevorzugt Cl, steht,
- R₃ für H oder -SO₂R₈ steht, worin R₈ für -CH₃, -NH₂ oder
- NH-CO-CH₂-CH₃ steht,
- R₄ für H oder ein Halogen, vorzugsweise I, Br, Cl oder F, und besonders bevorzugt F,
- R₅ für H oder CH₂-COOH,
- R₆ für H oder CH₃,
- R₇ für H oder ein Halogen, vorzugsweise I, Br, Cl oder F, und besonders bevorzugt Cl,
- X und Y unabhängig voneinander für N oder CH stehen,
- A für N oder C,
- B für N, CH₂ oder C-CH₃,
- D für O oder C-CF₃,
- E für N, CH oder C=O,
oder ein pharmazeutisch annehmbares Salz dieses Inhibitors, gegebenenfalls zusammen mit einem oder mehreren pharmazeutisch annehmbaren Exzipienten,
wobei die pharmazeutische Zubereitung weder eine Verbindung der folgenden allgemeinen Formel (VI) enthält, noch ein pharmazeutisch annehmbares Salz davon: 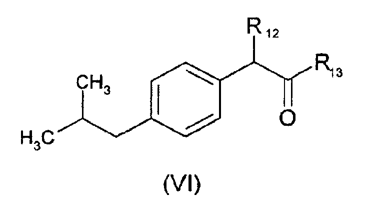
- in der R₁₂ für H oder eine Alkyl-Gruppe mit 1 bis 6 Kohlenstoffatomen steht,
- R₁₃ für eine Gruppe OR₁₄ oder NR₁₅R₁₆,
- R₁₄ für H, eine Alkyl-Gruppe mit 1 bis 6 Kohlenstoffatomen, eine Aryl-Gruppe mit 6 bis 10 Kohlenstoffatomen, oder eine Aralkyl- oder Alkaryl-Gruppe mit 7 bis 20 Kohlenstoffatomen,
- R₁₅ und R₁₆, identisch oder verschieden, für H, OH, eine Alkyl-Gruppe mit 1 bis 6 Kohlenstoffatomen, eine Aryl-Gruppe mit 6 bis 10 Kohlenstoffatomen, oder eine Aralkyl- oder Alkaryl-Gruppe mit 7 bis 20 Kohlenstoffatomen stehen.

2. Pharmazeutische Zubereitung nach Patentanspruch 1, in der die Verbindung der Formel (I) einer der folgenden Formeln (II), (III) und (IV) entspricht:

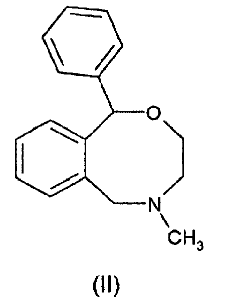

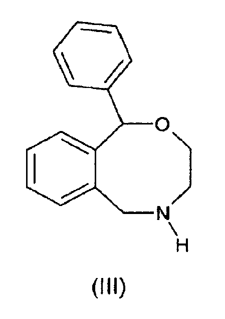

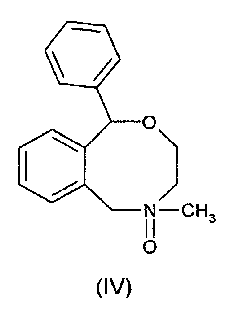

3. Pharmazeutische Zubereitung nach Patentanspruch 1 oder 2, in der der selektive Inhibitor von COX-2 aus der Gruppe ausgewählt wurde, bestehend aus Celecoxib, Parecoxib, Valdecoxib, Rofecoxib und Etoricoxib.

4. Pharmazeutische Zubereitung nach einem der Patentansprüche 1 bis 3, umfassend:
a) Nefopam-Chlorhydrat und
b) Celecoxib.

5. Pharmazeutische Zubereitung nach einem der Patentansprüche 1 bis 4, eine Einheitsdosis von 1 mg bis 120 mg der Verbindung der Formel (I) oder eines pharmazeutisch annehmbaren Salzes davon umfassend.

6. Pharmazeutische Zubereitung nach einem der Patentansprüche 1 bis 5, eine Einheitsdosis von 10 mg bis 1000 mg des selektiven Inhibitors von COX-2 oder eines pharmazeutisch annehmbaren Salzes davon enthaltend.

7. Pharmazeutische Zubereitung nach einem der Patentansprüche 1 bis 6, für eine orale oder lokale Verabreichung oder Verabreichung durch Injektion geeignet.

8. Pharmazeutische Zubereitung nach einem der Patentansprüche 1 bis 7 zur Herbeiführung von Schmerzunempfindlichkeit oder zu ihrem Gebrauch zur Vorbeugung oder Behandlung von Schmerzen.

9. Verbindung der allgemeinen Formel (I), oder pharmazeutisch annehmbares Salz davon, nach einem der Patentansprüche 1, 2 und 4, kombiniert mit
einem selektiven Inhibitor von COX-2 oder einem pharmazeutisch annehmbaren Salz davon, nach einem der Patentansprüche 1, 3 und 4,
zu ihrem Gebrauch als Medikament,
wobei die Verbindung der allgemeinen Formel (I) und der selektive Inhibitor von COX-2 oder deren pharmazeutisch annehmbare Salze nicht mit einer Verbindung der Formel (VI), wie in Patentanspruch 1 definiert, oder einem pharmazeutisch annehmbaren Salz davon, kombiniert werden.

10. Verbindungen nach Patentanspruch 9 oder pharmazeutisch annehmbare Salze davon, wobei die Verbindung der Formel (I) oder ein pharmazeutisch annehmbares Salz davon in einer Einheitsdosis von 1 mg bis 120 mg verabreicht wird.

11. Verbindungen nach Patentanspruch 9 oder 10 oder pharmazeutisch annehmbare Salze davon, wobei der selektive Inhibitor von COX-2 oder ein pharmazeutisch annehmbares Salz davon in einer Einheitsdosis von 10 mg bis 1000 mg verabreicht wird.

12. Verbindungen nach einem der Patentansprüche 9 bis 11 oder pharmazeutisch annehmbare Salze davon, wobei die Verbindung der Formel (I) oder ein pharmazeutisch annehmbares Salz davon intravenös, oral oder intramuskulär verabreicht wird.

13. Verbindungen nach einem der Patentansprüche 9 bis 12 oder pharmazeutisch annehmbare Salze davon, wobei der selektive Inhibitor von COX-2 oder ein pharmazeutisch annehmbares Salz davon oral, lokal oder durch Injektion verabreicht wird.

14. Verbindungen nach einem der Patentansprüche 9 bis 13 oder pharmazeutisch annehmbare Salze davon zur Herbeiführung von Schmerzunempfindlichkeit oder zu ihrem Gebrauch zur Vorbeugung oder Behandlung von Schmerzen.

15. Produkte,
- mindestens eine Verbindung der allgemeinen Formel (I), oder ein pharmazeutisch annehmbares Salz davon, nach einem der Patentansprüche 1, 2 und 4, und
- mindestens einen selektiven Inhibitor von COX-2, oder ein pharmazeutisch annehmbares Salz davon, nach einem der Patentansprüche 1, 3 und 4
enthaltend, als Kombinationsprodukt zum gemeinsamen oder getrennten Gebrauch zur Herbeiführung von Schmerzunempfindlichkeit oder zur Vorbeugung oder Behandlung von Schmerzen, wobei die Produkte weder eine Verbindung der allgemeinen Formel (VI), wie in Patentanspruch 1 definiert, enthalten, noch ein pharmazeutisch annehmbares Salz davon.

16. Produkte nach Patentanspruch 15, eine Einheitsdosis von 1 mg bis 120 mg der Verbindung der Formel (I) oder eines pharmazeutisch annehmbaren Salzes davon enthaltend.

17. Produkte nach Patentanspruch 15 oder 16, eine Einheitsdosis von 10 mg bis 1000 mg des selektiven Inhibitors von COX-2 oder eines pharmazeutisch annehmbaren Salzes davon enthaltend.

18. Produkte nach einem der Patentansprüche 15 bis 17, in dem sich die Verbindung der allgemeinen Formel (I), oder ein pharmazeutisch annehmbares Salz davon, in einer zur intravenösen, oralen oder intramuskulären Verabreichung geeigneten Form befindet.

19. Produkte nach einem der Patentansprüche 15 bis 18, in dem sich der selektive Inhibitor von COX-2 oder ein pharmazeutisch annehmbares Salz davon in einer zur oralen oder lokalen Verabreichung oder zur Verabreichung durch Injektion geeigneten Form befindet.

## Claims

1. A pharmaceutical composition comprising as active substances:
a) at least one compound of the following general formula (I): 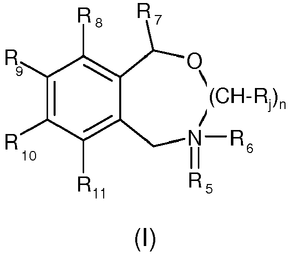 in which:
- R₅ represents O or no group;
- R₆ represents H or an alkyl group containing from 1 to 6 carbon atoms;
- n represents an integer from 2 to 4;
- j represents an integer in the range from 1 to n;
- Rⱼ, identical or different for each substituted carbon, represents H or an alkyl group containing from 1 to 6 carbon atoms;
- R₇ represents a phenyl group optionally substituted with one or more groups, which may be identical or different, selected from the group consisting of H, an alkyl group containing from 1 to 6 carbon atoms, an alkoxy group containing from 1 to 6 carbon atoms, a trifluoromethyl group, or a halogen atom;
- R₈, R₉, R₁₀, R₁₁, which may be identical or different, represent H, an alkyl group containing from 1 to 6 carbon atoms, an alkoxy group containing from 1 to 6 carbon atoms, a trifluoromethyl group, or a halogen atom;
or a pharmaceutically acceptable salt of this compound; and
b) at least one selective inhibitor of type 2 cyclooxygenase (COX-2) represented by the following formula (V): 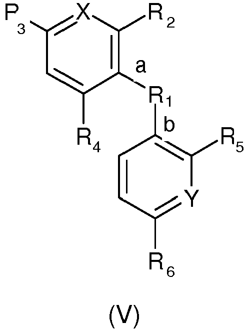 in which:
- R₁ is selected from the group consisting of 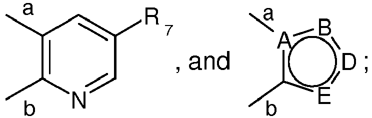
- a and b make it possible to determine the orientation of R₁;
- R₂ represents H or a halogen, preferably I, Br, Cl or F, and more preferably Cl;
- R₃ represents H or -SO₂R₈, where R₈ represents -CH₃, -NH₂ or -NH-CO-CH₂-CH₃;
- R₄ represents H or a halogen, preferably I, Br, Cl or F, and more preferably F;
- R₅ represents H or CH₂-COOH;
- R₆ represents H or CH₃;
- R₇ represents H or a halogen, preferably I, Br, Cl or F, and more preferably Cl;
- X and Y represent independently N or CH;
- A represents N or C;
- B represents N, CH₂ or C-CH₃;
- D represents O, or C-CF₃;
- E represents N, CH or C=O.
or a pharmaceutically acceptable salt of this inhibitor;
optionally together with one or more pharmaceutically acceptable excipients,
the pharmaceutical composition not comprising a compound of the following general formula (VI), or a pharmaceutically acceptable salt thereof: 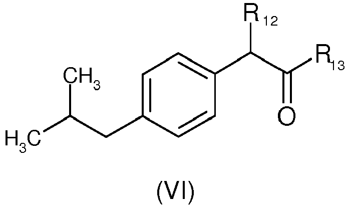
- R₁₂ representing H or an alkyl group containing from 1 to 6 carbon atoms;
- R₁₃ representing a OR₁₄ or NR₁₅R₁₆ group;
- R₁₄ representing H, an alkyl group containing from 1 to 6 carbon atoms, an aryl group containing from 6 to 10 carbon atoms, or an aralkyl or alkaryl group containing from 7 to 20 carbon atoms;
- R₁₅ and R₁₆, which may be identical or different, representing H, OH, an alkyl group containing from 1 to 6 carbon atoms, an aryl group containing from 6 to 10 carbon atoms, or an aralkyl or alkaryl group containing from 7 to 20 carbon atoms.

2. The pharmaceutical composition according to claim 1, wherein the compound of formula (I) is represented by one of the following formulae (II), (III) and (IV):

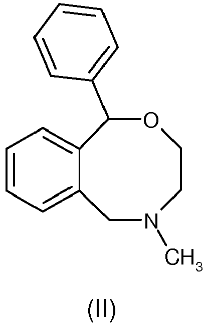

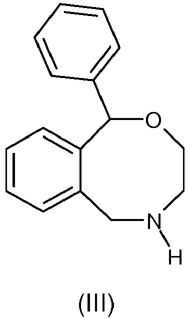

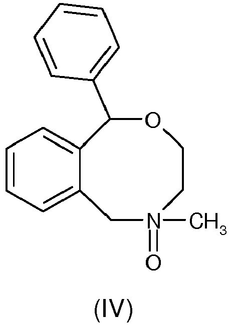

3. The pharmaceutical composition according to claim 1 or 2, wherein the COX-2-selective inhibitor is selected from the group consisting of celecoxib, parecoxib, valdecoxib, rofecoxib, and etoricoxib.

4. The pharmaceutical composition according to any of claims 1 to 3, comprising:
a) nefopam hydrochloride, and
b) celecoxib.

5. The pharmaceutical composition according to any of claims 1 to 4, comprising a unit dose of from 1 mg to 120 mg of the compound of formula (I) or of a pharmaceutically acceptable salt thereof.

6. The pharmaceutical composition according to any of clams 1 to 5, comprising a unit dose of from 10 mg to 1000 mg of the COX-2-selective inhibitor or of a pharmaceutically acceptable salt thereof.

7. The pharmaceutical composition according to any of claims 1 to 6, suitable for administration by the oral route, by injection or locally.

8. The pharmaceutical composition according to any of claims 1 to 7, for inducing analgesia or for its use in the prevention or treatment of pain.

9. A compound of the general formula (I), or a pharmaceutically acceptable salt thereof, as defined in any of claims 1, 2 and 4, in combination with
a COX-2-selective inhibitor, or a pharmaceutically acceptable salt thereof, as defined in any of claims 1, 3 and 4,
for their use as a medicament,
the compound of the general formula (I) and the COX-2-selective inhibitor, or their pharmaceutically acceptable salts, not being combined with a compound of formula (VI) as defined in claim 1, or a pharmaceutically acceptable salt thereof.

10. Compounds of pharmaceutically acceptable salts thereof according to claim 9, wherein the compound of formula (I), or a pharmaceutically acceptable thereof, is administered at a unit dose of from 1 mg to 120 mg.

11. Compounds of pharmaceutically acceptable salts thereof according to claim 9 or 10, wherein the COX-2-selective inhibitor, or a pharmaceutically acceptable salt thereof, is administered at a unit dose of from 10 mg to 1000 mg.

12. Compounds or pharmaceutically acceptable salts thereof according to any of claims 9 to 11, wherein the compound of formula (I), or a pharmaceutically acceptable salt thereof, is administered by the intravenous, oral, or intramuscular route.

13. Compounds or pharmaceutically acceptable salts thereof according to any of claims 9 to 12, wherein the COX-2-selective inhibitor, or a pharmaceutically acceptable salt thereof, is administered by the oral route, by injection or locally.

14. Compounds or pharmaceutically acceptable salts thereof according to any of claims 9 to 13, for inducing analgesia or for their use in the prevention or treatment of pain.

15. Products containing:
- at least one compound of the general formula (I), or a pharmaceutically acceptable salt thereof, as defined in any of claims 1, 3 and 4, and
- a COX-2-selective inhibitor, or a pharmaceutically acceptable salt thereof, as defined in any of claims 1, 3 and 4,
as a combination product for use together or separately for inducing analgesia or for preventing or treating pain,
the products comprising no compound of the general formula (VI) as defined in claim 1, or a pharmaceutically acceptable salt thereof.

16. Products according to claim 15, comprising a unit dose of from 1 mg to 120 mg of the compound of formula (I), or of a pharmaceutically acceptable salt thereof.

17. Products according to claim 15 or 16, comprising a unit dose of from 10 mg to 1000 mg of the COX-2-selective inhibitor, or of a pharmaceutically acceptable salt thereof.

18. Products according to any of claims 15 to 17, wherein the compound of formula (I), or a pharmaceutically acceptable salt thereof, is under a form suitable for being administered by the intravenous, oral, or intramuscular route.

19. Products according to any of claims 15 to 18, wherein the COX-2-selective inhibitor, or a pharmaceutically acceptable salt thereof, is under a form suitable for being administered by the oral route, by injection or locally.
